# EUROPEAN PATENT APPLICATION

(11) **EP 4 230 149 A1**
(43) Date of publication of application: **23.08.2023**
(21) Application number: 22157623.4
(22) Date of filing: 18.02.2022
(51) Int. Cl.: A61B 10/00, B01L 3/00

(54) **DEVICE FOR COLLECTING A SAMPLE, A KIT COMPRISING SUCH DEVICE AND A METHOD USING SUCH DEVICE OR KIT**

(71) Applicant: MolGen B.V., 3905 NR Veenendaal (NL)
(72) Inventor: MILLENAAR, Bas, 3941RN Doorn (NL); MENTINK, Chris, 6845AX Arnhem (NL)
(74) Representative: Simmons & Simmons LLP

(57) **Abstract**

Device for collecting a sample, comprising a container (3) having a first region (11) for receiving the sample (2) and a second region (12) containing a substance (4) for preserving or stabilizing the sample, and a barrier which, when active, is located between the first region (11) and the second region (12) so that the sample and the substance are kept separate and, when inactive, allows the sample to get into contact with the substance, characterized in that the device (1) also comprises a sample guiding part (6) attachable to the container, said sample guiding part forming the barrier so that, when the sample guiding part is attached to the container, the barrier is active and, when the sample guiding part is at least partially detached from the container, the barrier is inactive.

## Description

The present invention relates to a device for collecting a sample, a kit comprising such device and a method using such device or kit.

More particularly, the invention relates to a device for collecting a sample, such as an upper respiratory tract sample.

Lately, upper respiratory tract samples have been used in testing to identify people who are infected with SARS CoV-2, a coronavirus disease. The testing has been performed most commonly through polymerase chain reaction (PCR) or reverse transcriptase-polymerase chain reaction (RT-PCR) molecular assays to detect viral RNA from the samples.

Typically, upper respiratory tract samples used for such testing purposes are nasopharyngeal, oropharyngeal or nasal swabs. Although initially adopted as a preferred sampling technique, swab collections are uncomfortable, inherently risky for contamination and not well suited for repeat testing. They require a swab being inserted into the nose, which can cause irritation that could promote sneezing and coughing. Swabs are also associated with variable, inconsistent and false-negative test results due to the technical difficulties of taking a proper swab.

Alternatively, it has been shown that saliva samples can serve as upper respiratory tract samples for SARS-CoV-2 detection. Saliva is a non-invasive collection type which does not require a swab. Therefore, it allows for an easy and convenient sample collection.

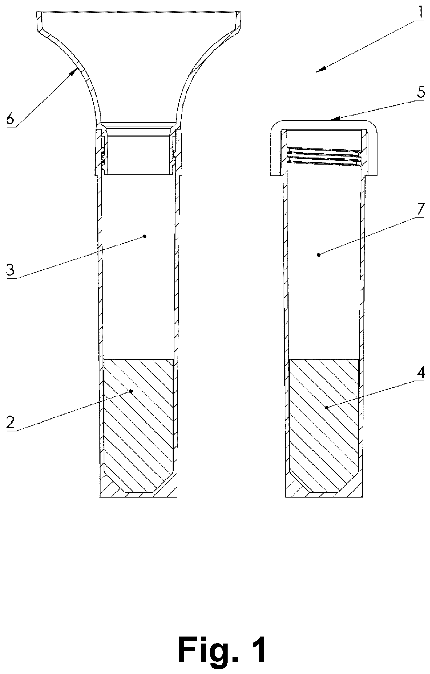

Devices for collecting saliva samples are known in the art. An example of such known device is shown in figure 1. This figure illustrates a device 1 suitable for collecting a saliva sample 2. The device 1 comprises two separate containers, namely a first container 3 for receiving the saliva sample 2 and a second container 7 containing a substance 4 for preserving or stabilizing the saliva sample 2. As the substance 4 is usually toxic, measures must be taken so as to avoid any contact between the user of the device 1 and the substance 4, or to avoid the user drinking the substance 4, as much as possible. In this case, this is accomplished by having the substance 4 contained in the container 7 which is separate from the container 3 which serves to receive the saliva sample 2. In this way, the risk of the user getting into contact with, or drinking the substance 4, while the saliva sample 2 is being collected, is reduced. The device 1 also comprises a cap 5 for closing the separate container 7.

In order to collect the saliva sample 2, the user of the device 1 can spit into the container 3. In order to properly guide the saliva sample 2 into the container 3, the device 1 comprises a sample guiding part 6 attachable to the container 3. In this case, the sample guiding part 6 comprises a funnel. Then, once the saliva sample 2 is collected in the container 3, the separate container 7 can be opened by removing the cap 5 and the substance 4 can be transferred from the separate container 7 to the container 3 in order for the substance 4 to get into contact with the saliva sample 2. Finally, the cap 5 can also be used to close the container 3 after the sample guiding part 6 is removed. However, this procedure can still be misinterpreted by the user, because s/he may drink the substance 4, or wash the content of the separate container 7, instead of transferring the substance 4 from the separate container 7 to the container 3. Therefore, self-collection of the saliva sample 2, meaning that the collection of the saliva sample 2 is performed without supervision of qualified personnel, remains difficult. Also, the handling of the device 1 is complex, because it contains many separate parts or components.

Another example of a known device for collecting a saliva sample is described in US 2017/0152545 A1 and more particularly illustrated in figures 10 and 11 thereof. The device shown in these figures comprises a container having a first region for receiving the saliva sample and a second region containing a substance for stabilizing or preserving the sample. The device also comprises a cap which is screwable to the container in order to close it. Furthermore, the device comprises a barrier which, when active, is located between the first region and the second region so as to keep the saliva sample and the substance separate and, when inactive, allows the sample to get into contact with the substance. The barrier is a sealing disc. The sealing disc is configured so that, when the cap is not screwed to the container, the barrier is active and, as the cap is screwed on, the sealing disc is pivoted, the barrier thereby being inactivated. Due to the presence of the barrier, the device is relatively safe, because it prevents the substance from escaping from the container, while the saliva sample is being collected.

However, the mechanism for inactivating the barrier is fairly complicated. It is also difficult to manufacture.

The objective of the present invention primarily is to provide an alternative device for collecting a sample, whereby, according to preferred embodiments, one or more of the aforementioned or other problems of the state of the art are solved. More particularly, the invention aims at providing a device that allows for a rapid and reliable self-collection of a sample or, in other words, unsupervised sample collection, enabling to increase the testing capacity. Another aim is to provide a device that is simpler and/or easier to manufacture than existing devices.

Thereto, the present invention according to a first independent aspect relates to a device for collecting a sample, comprising a container having a first region for receiving the sample and a second region containing a substance for preserving or stabilizing the sample, and a barrier which, when active, is located between the first region and the second region so as to keep the sample separate from the substance and, when inactive, allows the sample to get into contact with the substance, with the characteristic that the device also comprises a sample guiding part, for example comprising a funnel, which is attachable to the container, said sample guiding part forming the barrier, so that, when the sample guiding part is attached to the container, the barrier is active and, when the sample guiding part is at least partially detached from the container, the barrier is inactive. With this characteristic, the advantage is obtained that a safe self-collection of samples, such as saliva samples, is allowed, without the need for supervision of qualified personnel. Furthermore, the construction of the device is relatively simple, which allows for an easy manufacture.

In a particular embodiment, the barrier is less wide than the first region. Thereby, the advantage is obtained that the barrier can be easily moved, preferably in a translational manner, upwards and/or out of the container, whereby the sample is allowed to flow in a smooth manner past the barrier to get into contact with the substance.

More particularly, the first region may be wider than the second region. For example, the container may comprise an outer wall forming the first region and an inner wall forming the second region.

Alternatively, the first region may be approximately as wide as the second region, the regions preferably being connected by a narrower section. The narrower section may be capable of receiving the barrier when active. For example, the container may be hourglass-shaped. Such shape provides the advantage that it allows to reduce the overall width of the container, since a double wall construction or the like may be avoided.

In another embodiment, the barrier is configured so as to allow the sample to pass through it when the sample guiding part is being detached from the container. For that purpose, the barrier may comprise one or more one-way valves or the like. With this embodiment, the advantage is obtained that the overall width of the container can be reduced, for example making it possible to place the container in a well plate or the like.

In still another embodiment, the barrier is breakable and configured to break when the sample guiding part is being detached from the container, thereby allowing the sample to get into contact with the substance. For that purpose, the barrier may comprise one or more weakened portions, such as notches or the like. With this embodiment, the advantage is also obtained that the overall width of the container can be reduced.

The aforementioned embodiments may be combined.

The sample guiding part may comprise a stopper having the barrier. More particularly, the stopper may comprise an elongated portion with an end portion, which forms the barrier.

According to a second independent aspect, the present invention relates to a device for collecting a sample, comprising a container having a first region for receiving the sample and a second region containing a substance for preserving or stabilizing the sample, and a barrier which, when active, is located between the first region and the second region so that the sample and the substance are kept separate and, when inactive, allows the sample to get into contact with the substance, with the characteristic that the barrier comprises a pierceable seal configured so as to transition from active to inactive by being pierced by a sample guiding part attachable to the container. With this characteristic, the advantage is obtained that a safe self-collection of samples, such as saliva samples, is allowed, without the need for supervision of qualified personnel.

Furthermore, the construction of the device is relatively simple, which allows for an easy manufacture.

The pierceable seal may be made from a same material as the container. The pierceable seal may be a thin sheet of material. The pierceable seal may be attached to the container, for example by means of a press-fit, a glue and/or a heat treatment.

In a particular embodiment, the pierceable seal may be pierced by engaging the sample guiding part with the seal, for example by turning or pushing. The sample guiding part may comprise a sharp tip to pierce the seal.

According to a third independent aspect, the present invention relates to a device for collecting a sample, comprising a container having a first region for receiving the sample and a second region containing a substance for preserving or stabilizing the sample, and a barrier which, when active, is located between the first region and the second region so that the sample and the substance are kept separate and, when inactive, allows the sample to get into contact with the substance, with the characteristic that the barrier comprises a dissolvable seal configured so as to transition from active to inactive by dissolution. With this characteristic, the advantage is obtained that a safe self-collection of samples, such as saliva samples, is allowed, without the need for supervision of qualified personnel. Furthermore, the construction of the device is relatively simple, which allows for an easy manufacture.

Preferably, the dissolvable seal is configured so as to dissolve or start dissolving when getting into contact with the sample.

The rapidness of dissolution can be determined by several factors, such as the material of the seal, the thickness of the seal and/or any heating temperature that may be applied to the seal. For example, it has been found that amylase, which is present in a saliva sample, slowly dissolves a seal made of a starch substance. Such slowness may be beneficial in terms of the safety of the device. As another example, the seal may contain a retarding agent, so as to slow down the dissolution process.

The dissolvable seal may be made of any suitable material. Examples include: polyvinyl alcohol (PVA), a gel substance, a starch substance, or a gelatine (hard or soft) or non-gelatine shell.

According to a fourth independent aspect, the present invention relates to a device for collecting a sample, comprising a container for receiving the sample and containing a substance for preserving or stabilizing the sample, with the characteristic that the device also comprises a one-way barrier so as to allow the sample but prevent the substance to flow through it. With this characteristic, the advantage is obtained that a safe self-collection of samples, such as saliva samples, is allowed, without the need for supervision of qualified personnel. Furthermore, the construction of the device is relatively simple, which allows for an easy manufacture. Also, the sample can immediately get into contact with the substance upon collection.

In an example, the barrier may be a one-way valve. In another example, the barrier may be permeable by the sample but impermeable by the substance.

The barrier may be integrated into the container. Alternatively, the device may also comprise a collector aid, the barrier being integrated into the collector aid. The collector aid may be a straw.

According to a fifth independent aspect, the present invention relates to a device for collecting a sample, comprising a container for receiving the sample and containing a substance for preserving or stabilizing the sample, with the characteristic that the substance comprises a solid, which is prevented from escaping from the container. For example, the solid may be locked or trapped in the container, or it may be attached to the container, or in any other way prevented from escaping from the container. With this characteristic, the advantage is obtained that a safe self-collection of samples, such as saliva samples, is allowed, without the need for supervision of qualified personnel. Furthermore, the construction of the device is relatively simple, which allows for an easy manufacture.

The device may contain a grid, which locks or traps the solid in the container. The advantage of a grid is that the sample may flow through it and get into contact with the substance, whereas the substance cannot escape from the container or device. The grid may be mounted or pressed in the container. In another example, the device may contain a seal or coating which locks or traps the solid in the container. The seal or coating may dissolve or start dissolving when brought into contact with the sample, or may be permeably by the sample. In still another example, the solid may be treated so as to stick to the container.

The solid may be selected from one or more of the following: a pill, a granulate, a powder and a coating.

The following features that are described may be combined with any of the aforementioned and other aspects that are part of the present invention.

Preferably, the device comprises a sample guiding part attachable to the container. A sample guiding part assists in guiding the sample, such as saliva sample, into the container. It makes the collection process easier. The sample guiding part may comprise a funnel. The capillary action created by the funnel promotes guidance of the sample into the container. The sample guiding part may also be a collector aid, such as a straw.

The sample guiding part may be attached to the container in any suitable way. For example, they can be attached to each other by screwing or clicking or injection moulding.

Preferably, the sample guiding part is attachable to the container with tension. For example, the sample guiding part and the tube may be clamped to each other in the attached state.

The sample guiding part and/or the container may be made of plastic. In a particular embodiment, the sample guiding part may be made of a different plastic than the container. In this way both parts can be tuned to their respective needs. For example, the sample guiding part can be made of a plastic with hydrophobic properties making it easier for the sample to be guided into the container. The sample guiding part may also in itself be made from different plastics.

The sample guiding part and/or the container may be made of one or more of the following plastics: acrylics, epoxies, polyethylene, polystyrene, polyvinylchloride, polytetrafluorethylene, polydimethylsiloxane, polyesters, and polyurethanes.

The sample guiding part may be provided with a coating. The sample guiding part may be coated to attain certain properties, such as to inherit positive sample flow properties, to promote lysis, or to perform post-collection steps, such as an extraction or cleaning step, or at least a part thereof.

The sample guiding part may also be shaped so as to promote guidance of the sample into the container. The sample guiding part may for example be hourglass-shaped or have round shape.

The container may have an inner wall and an outer wall. The substance may be contained within the inner wall. Thereby, the advantage is obtained that the resulting mix of the sample and the substance may reach a higher level in the container, by which it is made easier to extract the mixture from the container and extract more mixture volume, by means of for example small-size equipment such as a pipette or the like. Also, the outer wall of the container maintains easy handling in for example robotics that may be used in further processing.

The container may have any shape. Preferably, the container is a tube, or a vial.

Preferably but not limited, the container has a total volume capacity of 5 ml or less. The total volume capacity refers to the volume capacity for the sample and the substance when combined.

Preferably, the container is shaped so it can be placed in a well plate. A well plate is also called a microplate, microwell, microtiter or multiwell plate. So instead of having to transfer the sample from the container to the well plate, the container itself can be placed in the well plate. This is simple and efficient.

The container can be placed in the well plate for various purposes. For example it can be placed therein for performing a step of purifying, isolating or extracting RNA/DNA contained in the sample. This step may be performed using magnetic bead-based extraction, for example using tip combs configured to work together with the well plate. For that purpose, or for other reasons, it may be beneficial to have a container that is lockable in the well plate. Thereby, the risk of the container being pulled out of the well plate by the tip coms is reduced or even eliminated. Any type of locking system, mechanical or otherwise, may be used. For example, a click system can be used, whereby the container can be clicked to the well plate.

Preferably, the container is lockable in a well plate. Thereby, the risk of the container being mispositioned or misplaced is reduced or even eliminated. As described above, any type of locking system may be used for this purpose.

A well plate can have 96, 384 or 1536 wells, however is not limited to the amount of wells. The well plate preferably has 96 wells, because it can be used manually by a laboratory technician or researcher. The wells of a well plate may hold several millimetres of liquid, preferably about 2ml.

The device is preferably configured so that the sample is self-collectable. More particularly, the device may serve to self-collect small-volume samples, that is samples in the range of 0.3 ml to 5 ml, preferably samples of about 2 ml.

The device may comprise a cap for closing the container. Preferably, the cap is designed so as to provide a liquid-tight and/or airtight seal for the container.

In a particular embodiment, the device has a scannable label, such as a barcode or a QR code. The scannable label may be attached to the container, such as to the side or the bottom of the container. The scannable label may be readable by a camera, such as the camera of a mobile device or a smartphone. A scannable label may improve the sample traceability or may facilitate automated sample processing necessary to support large scale testing.

By means of the scannable label, the device may be registered. It may be registered in connection with the identity of a user. Alternatively, it may be registered in connection with a video image of the collection process or other non-identifiable means. This allows the registration process or any other steps in the testing procedure to be performed by respecting privacy, such as by Privacy by Design. The same means used for registration can also be used to receive a test result derived from the collected sample. The means may for example be a software application.

Preferably, the device has a scale marking for indicating the sample volume required. In this way, it can be easily determined by the user whether enough sample volume has been collected. In the case of a saliva sample, about 2ml is usually sufficient. The scale marking promotes the self-collectability of the sample.

The device may comprise a collector aid, such as a straw. The collection aid may promote providing true sample, such as saliva sample, and minimize potential aerosolization. In the case of a saliva sample, it may also avoid accidental collection of sputum and/or remnants from food or drinks, which can interfere with sample processing or inhibit certain reactions.

The device may comprise a second container containing or fillable with a second substance. The second container may be separate from the (first) container, or not. The second substance is preferably a rinsing and/or gargling liquid, such as water. In this way, especially in the case of a saliva sample, more sample can be collected by the device. The second container may be accompanied with a pipette or the like to extract the second substance. A predetermined amount of the second substance may be present in the second container.

The device of the present invention is especially suitable to collect a saliva sample. However, it may be used to collect any type of sample. For example, the sample may be a biological sample. The sample may be selected from one or more of the following: sputum, saliva, milk, blood, urine and faeces.

The container is preferably prefilled with the substance, so that the substance is already present in the container when it has to be used.

The substance preferably comprises a composition for stabilizing or preserving the sample, or certain components of the sample. For example, it may serve to stabilize or preserve nucleic acids, proteins, peptides, toxins, chitins, fatty acids, glycogens, DNA, RNA, or other components that could be analysed or measured. Preferably, the substance serves to preserve or stabilize nucleic acids, RNA or DNA. The substance may also serve to inactivate a virus that may be contained in the sample.

The substance may be liquid or solid.

The substance may for example include TBE, TE, or PBS. The substance may comprise additives such as Triton-X-100, Tween 20, or NP-40.

In a particular embodiment, the substance may be configured to colour when getting into contact with the sample. For example, the substance may be configured to colour upon a change in pH value when brought into contact with the sample.

According to a sixth independent aspect, the present invention also relates to a kit for collecting a sample, comprising a device as described herein. It may also comprise other components, such as a shipper box and/or instructions how to accomplish the sample collection. The shipper box may be an envelope or mailing container for shipment of the collected sample to a laboratory or facility, such as for RNA/DNA isolation and analysis. The components of the kit are preferably packed or suited to be packet together.

According to a seventh independent aspect, the present invention also relates to a method for collecting a sample, the method comprising the step of collecting a sample, such as a saliva sample, using the device or kit as described herein.

The present invention may be particularly advantageous to be used in SARS CoV-2 detection, given the urgent need to upscale the testing capacity. Its use is however not limited hereto. The present invention has a wide range of applications including RNA isolation and evaluation of RNA virus infection, gene detection, viral, bacterial, fungus, cytology sample processing, virology cell culture, ELISA, molecular diagnostic detection and saliva extraction of microorganisms. The present invention may be used for the detection of viral and bacterial targets.

The sample that is collected with the present invention can be analysed. It can for example be analysed to detect viral RNA/DNA or to identify infection with SARS CoV-2, the novel coronavirus. The analysis can be performed using conventional methods including the steps of placing the sample in a well plate, of purifying, isolating or extracting RNA/DNA contained in the sample, of amplifying RNA/DNA, for example using PCR, and/or of analysing the sample, for example to identify or detect viral RNA/DNA or infection with SARS CoV-2.

To better illustrate the features of the invention, certain preferred embodiments are described below, as examples that are not to be understood restrictively, with reference to the accompanying drawings, wherein:
- figure 1 shows a known device for collecting a sample;
- figure 2 shows a preferred embodiment of a device according to the present invention in collection mode;
- figure 3 shows the same embodiment as in figure 2 with the sample collected;
- figure 4 shows a variant of the device of figures 2 and 3 according to the present invention with a breakable barrier;
- figure 5 shows another preferred embodiment of a device according to the present invention with a dissoluble seal;
- figure 6 shows a further preferred embodiment of a device according to the present invention with a one-way barrier;
- figure 7 shows still a further preferred embodiment of a device according to present the invention with a solid substance;
- figure 8 shows a variant of figure 7 according to the present invention; and
- figure 9 shows a device according to the present invention which is placed in a well plate.

Figure 1 shows a known device for collecting a sample. This device has already been described in the introductory portion of the present disclosure.

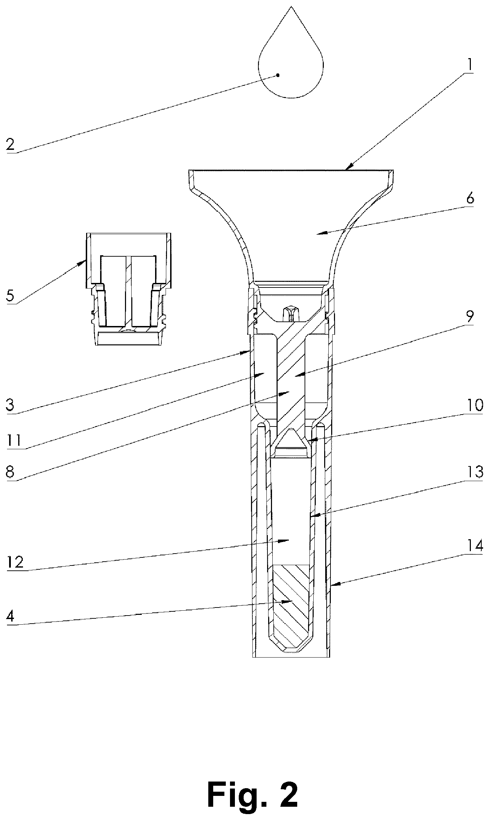

Figure 2 shows a preferred embodiment of a device for collecting a sample according to the present invention. More particularly, the device 1 is suitable for collecting a saliva sample 2. Figure 2 shows the device 1 in collection mode. In other words, it is ready to collect a saliva sample from a user.

The device 1 comprises a container 3. More particularly, the container 3 is a tube.

The device 1 also comprises a cap 5 for closing the container 3. For example, the container 3 can be closed with the cap 5 when the saliva sample 2 is collected. The cap 5 may be screwable or clickable to the container 3.

Furthermore, the device 1 comprises a sample guiding part 6 attachable to the container 3. More particularly, the sample guiding part 6 comprises a funnel.

The container 3 has a first region 11 for receiving the saliva sample 2. The container 3 also has a second region 12 which contains a substance 4 for preserving or stabilizing the saliva sample 2. In the case of figure 2, the container 3 is prefilled with the substance 4. As such, a user does not need to fill the container 3 with the substance 4, thereby reducing safety risks.

The device 1 comprises a barrier 10. When active, the barrier 10 is located between the first region 11 and the second region 12, so as to keep the saliva sample 2 and the substance 4 separate. This is the case in figure 2. The device 1 is in collection mode. As can be seen from figure 2, the substance 4 is prevented from escaping the container 3, or at least counteracted, thereby avoiding or reducing the risk that a user gets into contact with, or drinks the substance 4 when the saliva sample 2 is being collected, for example in the course of the spitting process.

The barrier 10 is formed by the sample guiding part 6. In figure 2, the sample guiding part 6 is attached to the container, the barrier 10 thereby being active. More particularly, the sample guiding part 6 comprises a stopper 8. The stopper 8 comprises an elongated portion 9 with an end portion 10, which forms the barrier.

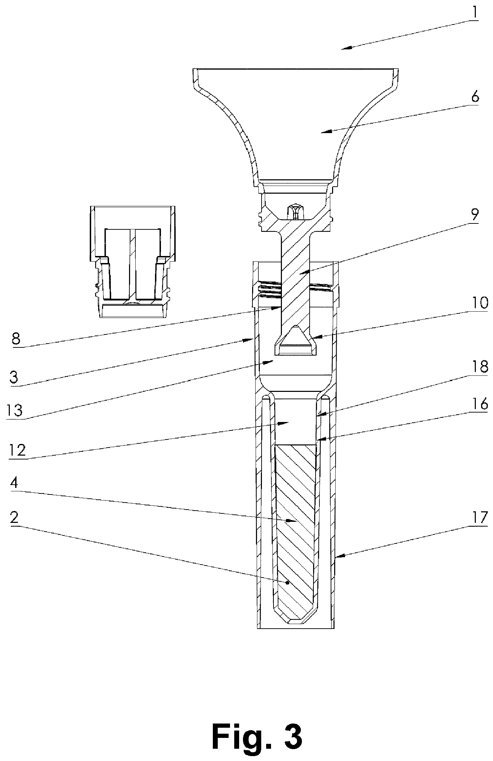

In figure 3, the same device 1 is shown as in figure 2. However, figure 3 shows the barrier 10 in the inactive state. Then, the barrier 10 allows the saliva sample 2 to get into contact with the substance 4. In this case, the sample guiding part 6 is being detached from the container 3, thereby inactivating the barrier 10.

As can be seen from figure 3, the barrier 10 is less wide than the first region 11. Thereby, the barrier 10 can be moved upwards in a translational manner, while the saliva sample 2 is able to flow past the barrier 10 towards the second region 12. The first region 11 is also wider than the second region 12.

Furthermore, the container 3 comprises an outer wall 14 forming the first region 11 and an innerwall 13 forming the second region 12. In this manner, the advantage is obtained that the container, because of the outer wall 14, can be easily handled in robotics that may be used in a further processing. By means of the inner wall 13, the advantage is obtained that the sample volume will lie at a higher level. That makes it easier for equipment such as a pipette to extract sample volume from the container 3. As a result, more sample volume may be obtained. That is beneficial for testing purposes.

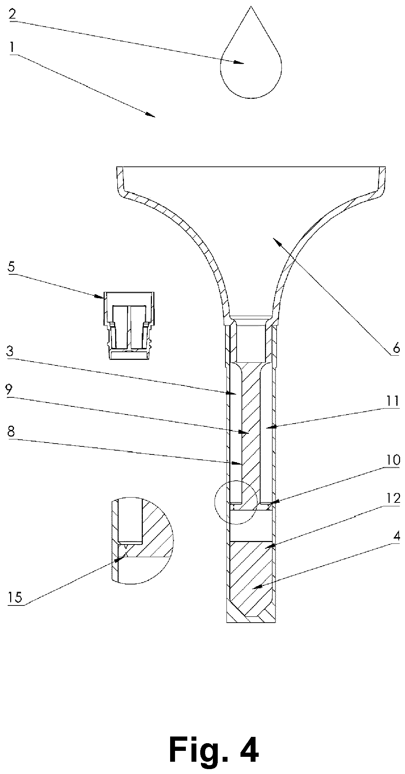

Figure 4 shows a variant of the device of figures 2 and 3. The device 1 is shown in the collection mode. The barrier 10 is active so as to keep the sample 2 and the substance 4 separate. The sample guiding part 6 is attached to the container 3, thereby the barrier 10 being active.

The barrier 10 is breakable and configured to break when the sample guiding part 6 is being detached from the container 3. For example, the barrier 10 may comprise a click system or other locking system so as to lock the barrier 10 in the container 3. When the sample guiding part 6 is being detached, a force is exerted in order to overcome the locking force which tries to keep the barrier locked. Eventually, the force may be big enough to break the locking system, by which also the barrier 10 is broken. As a result, the barrier 10 is rendered inactive, thereby allowing the sample 2 to get into contact with the substance 4. In order to allow the barrier to break more easily, the barrier may contain one or more weakened portions 15.

An advantage of the device 1 of figure 4 is that the width of the container 3 may be reduced. For example, no double wall construction or the like is needed for this variant. More particularly, the container 3 may be easily shaped or sized so it can be placed or positioned in a well plate. In this way, no transfer is needed of the sample 2 from the container 3 to a well plate for a possible further processing step.

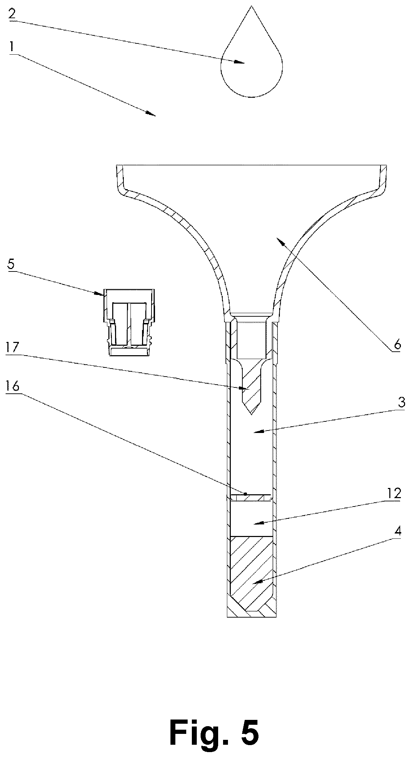

Figure 5 shows another preferred embodiment of a device for collecting a sample according to the present invention. The device 1 is shown in the collection mode. The barrier 16 is active so as to keep the sample 2 and the substance 4 separate from each other.

More particularly, the barrier 16 comprises a dissolvable seal. The dissolvable seal is configured so as to transition from active to inactive by dissolution. Preferably, the dissolvable seal is configured so as to dissolve or start dissolving when getting into contact with the sample 2.

The device 1 also comprises a guiding aid 17. More particularly, the guiding aid 17 is located at the bottom of the sample guiding part 6 in order to assist in guiding the sample 2 downwards.

An advantage of the device 1 of figure 5 is that it allows the width of the container 3 to be reduced. This may be advantageous in a further processing.

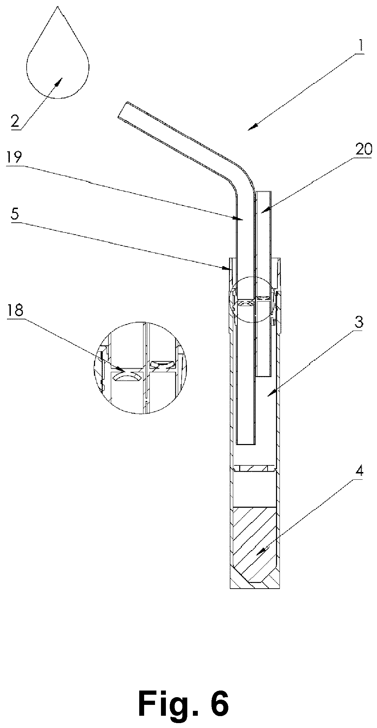

Figure 6 shows a further preferred embodiment of a device for collecting a sample according to the invention. The device 1 is shown in the collection mode.

The device 1 comprises a one-way barrier 18 so as to allow the sample 2 but prevent the substance 4 to flow through it. More particularly, the barrier 18 is integrated into a collector aid 19 of the device 1. In this example of figure 6, the collector aid 19 is a straw.

The device 1 also comprises a means 20 for allowing pressure to escape from the container 3.

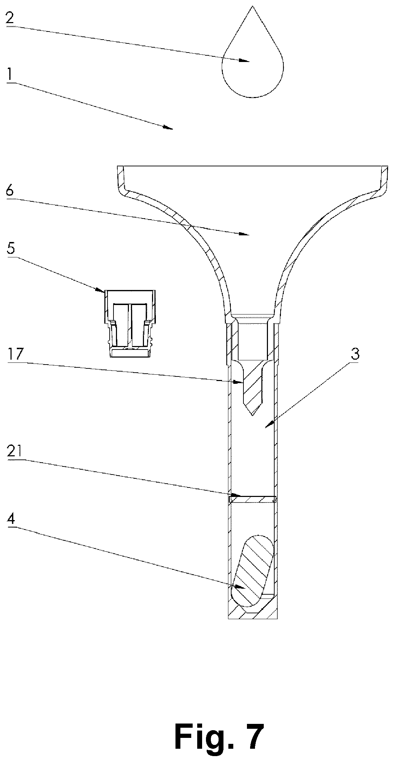

Figure 7 shows still a further preferred embodiment of a device for collecting a sample according to the invention. The device 1 is shown in the collection mode.

More particularly, the substance 4 comprises a solid. The solid is locked or trapped in the container 3. Especially, the device comprises a grid 21, which prevents the substance 4 from escaping from the container 3. The sample 2 may flow through the grid 21 so as to get into contact with the substance 4.

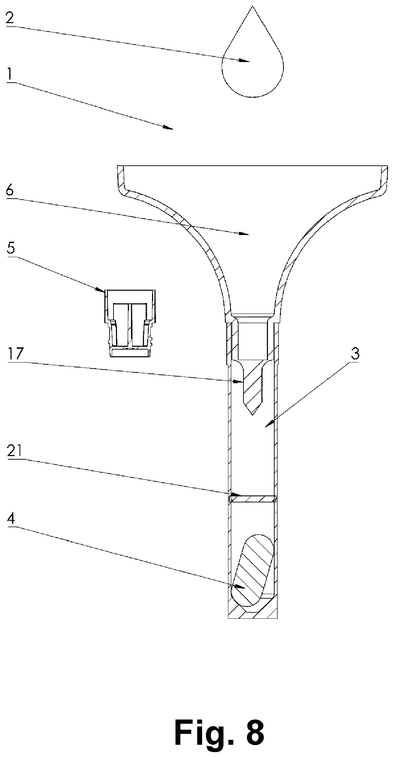

Figure 8 shows a variant of figure 7 according to the present invention. More particularly, the substance 4 comprises a solid, which is locked in the container 3 by a coating 22. The coating 22 may dissolve or start dissolving when getting into contact with the sample 2.

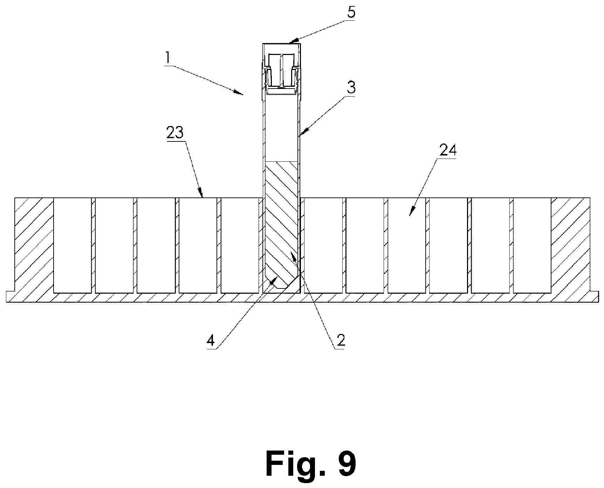

Figure 9 shows a container 3 that is shaped so it can be placed in a well plate 23. More particularly, the container 3 fits into one of the holes 24 of the well plate 23.

The present invention also relates to the items as listed below. These items describe preferred embodiment of the present invention. The reference numbers or letters contained in these items are not to be interpreted as limiting but as used for explanatory purposes.
1.- Device for collecting a sample, comprising a container (3) having a first region (11) for receiving the sample (2) and a second region (12) containing a substance (4) for preserving or stabilizing the sample, and a barrier which, when active, is located between the first region (11) and the second region (12) so that the sample and the substance are kept separate and, when inactive, allows the sample to get into contact with the substance, characterized in that the device (1) also comprises a sample guiding part (6) attachable to the container, said sample guiding part forming the barrier so that, when the sample guiding part is attached to the container, the barrier is active and, when the sample guiding part is at least partially detached from the container, the barrier is inactive.
2.- Device according to item 1, wherein the barrier is less wide than the first region.
3.- Device according to item 1 or 2, wherein the first region is wider than the second region.
4.- Device according to any of the preceding items, wherein the container comprises an outer wall (14) forming the first region and an inner wall (13) forming the second region.
5.- Device according to item 1 or 2, wherein the first region is approximately as wide as the second region, the regions preferably being connected by a narrower section.
6.- Device according to item 5, wherein the container is hourglass-shaped.
7.- Device according to any of the preceding items, wherein the barrier is configured so as to allow the sample to pass through it when the sample guiding part is being detached from the container.
8.- Device according to any of the preceding items, wherein the barrier is breakable and configured to break when the sample guiding part is being detached from the container, thereby allowing the sample to get into contact with the substance.
9.- Device according to item 8, wherein the barrier comprises one or more weakened portions (15).
10.- Device according to any of the preceding items, wherein the sample guiding part comprises a stopper (8) having the barrier.
11.- Device according to item 10, wherein the stopper comprises an elongated portion (9) with an end portion (10), which forms the barrier.
12.- Device for collecting a sample, comprising a container (3) having a first region (11) for receiving the sample (2) and a second region (12) containing a substance (4) for preserving or stabilizing the sample, and a barrier which, when active, is located between the first region (11) and the second region (12) so that the sample and the substance are kept separate and, when inactive, allows the sample to get into contact with the substance, characterized in that the barrier comprises a pierceable seal configured so as to transition from active to inactive by being pierced by a sample guiding part (6) attachable to the container.
13.- Device for collecting a sample, comprising a container (3) having a first region (11) for receiving the sample (2) and a second region (12) containing a substance (4) for preserving or stabilizing the sample, and a barrier which, when active, is located between the first region (11) and the second region (12) so that the sample and the substance are kept separate and, when inactive, allows the sample to get into contact with the substance, characterized in that the barrier comprises a dissolvable seal (16) configured so as to transition from active to inactive by dissolution.
14.- Device according to item 13, wherein the dissolvable seal is configured so as to dissolve or start dissolving when getting into contact with the sample.
15.- Device for collecting a sample, comprising a container (3) for receiving the sample (2) and containing a substance (4) for preserving or stabilizing the sample, characterized in that the device also comprises a one-way barrier (18) so as to allow the sample but prevent the substance to flow through it.
16.- Device according to item 15, wherein the barrier is integrated into or located in the container.
17.- Device according to item 15, wherein the device also comprises a collector aid (19) and wherein the barrier is integrated into or located in the collector aid.
18.- Device for collecting a sample, comprising a container (3) for receiving the sample (2) and containing a substance (4) for preserving or stabilizing the sample, characterized in that the substance comprises a solid, which is prevented from escaping from the container, preferably by being locked or trapped in the container.
19.- Device according to item 18, wherein the device comprises a grid (21) or coating (22) locking or trapping the solid in the container.
20.- Device according to item 18 or 19, wherein the solid is selected from one or more of the following: a pill, a granulate, a powder and a coating.
21.- Device according to any of the preceding items, wherein the device comprises a sample guiding part attachable to the container.
22.- Device according to any of the preceding items, wherein the container has an inner wall and an outer wall, the substance preferably being contained within the inner wall.
23.- Device according to any of the preceding items, wherein the container is a tube or a vial.
24.- Device according to any of the preceding items, wherein the container has a total volume capacity of 10 ml or less, preferably of 5 ml or less.
25.- Device according to any of the preceding items, wherein the container is shaped so it can be placed or positioned in a well plate.
26.- Device according to any of the preceding items, wherein the device is configured so that the sample is self-collectable.
27.- Device according to any of the preceding items, wherein the device is configured so as to collect small-volume samples, i.e. samples in the range of 0.3 ml to 5 ml, preferably samples of about 2ml.
28.- Device according to any of the preceding items, wherein the device comprises a cap for closing the container.
29.- Device according to any of the preceding items, wherein the device has a scannable label, such as a barcode.
30.- Device according to any of the preceding items, wherein the device has a scale marking for indicating the sample volume required.
31.- Device according to any of the preceding items, wherein the device comprises a collector aid such as a straw.
32.- Device according to any of the preceding items, wherein the device comprises a second container containing or fillable with a second substance, such as a rinsing and/or gargling liquid, for example water.
33.- Device according to any of the preceding items, wherein the sample is a saliva sample.
34.- Device according to any of the preceding items, wherein the container is prefilled with the substance.
35.- Device according to any of the preceding items, wherein the substance is a liquid or solid.
36.- Kit for collecting a sample, comprising a device (1) according to any of the preceding items.
37.- Method for collecting a sample, comprising the step of collecting a sample using the device according to any of the items 1 to 35 and/or the kit according to item 36.

The present invention is in no way limited to the embodiments described above, but such construction machines, methods, power plants and apparatus may be realised according to different variants without going beyond the scope of the present invention.

## Claims

1. Device for collecting a sample, comprising a container (3) having a first region (11) for receiving the sample (2) and a second region (12) containing a substance (4) for preserving or stabilizing the sample, and a barrier which, when active, is located between the first region (11) and the second region (12) so that the sample and the substance are kept separate and, when inactive, allows the sample to get into contact with the substance, **characterized in that** the device (1) also comprises a sample guiding part (6) attachable to the container, said sample guiding part forming the barrier so that, when the sample guiding part is attached to the container, the barrier is active and, when the sample guiding part is at least partially detached from the container, the barrier is inactive.

2. Device according to claim 1, wherein the barrier is less wide than the first region.

3. Device according to claim 1 or 2, wherein the first region is wider than the second region.

4. Device according to any of the preceding claims, wherein the container comprises an outer wall (14) forming the first region and an inner wall (13) forming the second region.

5. Device according to any of the preceding claims, wherein the barrier is breakable and configured to break when the sample guiding part is being detached from the container, thereby allowing the sample to get into contact with the substance.

6. Device according to claim 5, wherein the barrier comprises one or more weakened portions (15).

7. Device according to any of the preceding claims, wherein the sample guiding part comprises a stopper (8) having the barrier.

8. Device according to claim 7, wherein the stopper comprises an elongated portion (9) with an end portion (10), which forms the barrier.

9. Device for collecting a sample, comprising a container (3) having a first region (11) for receiving the sample (2) and a second region (12) containing a substance (4) for preserving or stabilizing the sample, and a barrier which, when active, is located between the first region (11) and the second region (12) so that the sample and the substance are kept separate and, when inactive, allows the sample to get into contact with the substance, **characterized in that** the barrier comprises a dissolvable seal (16) configured so as to transition from active to inactive by dissolution.

10. Device according to claim 9, wherein the dissolvable seal is configured so as to dissolve or start dissolving when getting into contact with the sample.

11. Device for collecting a sample, comprising a container (3) for receiving the sample (2) and containing a substance (4) for preserving or stabilizing the sample, **characterized in that** the substance comprises a solid, which is prevented from escaping from the container, preferably by being locked or trapped in the container.

12. Device according to claim 11, wherein the device comprises a grid (21) or coating (22) locking or trapping the solid in the container.

13. Device according to claim 11 or 12, wherein the solid is selected from one or more of the following: a pill, a granulate, a powder and a coating.

14. Kit for collecting a sample, comprising a device (1) according to any of the preceding claims.

15. Method for collecting a sample, comprising the step of collecting a sample using the device according to any of the claims 1 to 13 and/or the kit according to claim 14.
